# EUROPÄISCHE PATENTSCHRIFT

(11) **EP 0 906 289 B1**
(45) Veröffentlichungstag und Bekanntmachung des Hinweises auf die Patenterteilung: **30.10.2002**
(21) Anmeldenummer: 97925052.9
(22) Anmeldetag: 06.06.1997
(51) Int. Cl.: C07D 239/30

(54) **VERFAHREN ZUR HERSTELLUNG VON 4,5,6-TRICHLOR- UND 2,4,5,6-TETRACHLORPYRIMIDIN**
METHOD OF PREPARING 4,5,6-TRICHLORO- AND 2,4,5,6-TETRACHLOROPYRIMIDINE
PROCEDE DE PREPARATION DE 4,5,6-TRICHLORO- ET 2,4,5,6-TETRACHLOROPYRIMIDINE

(30) Priorität: 10.06.1996 DE 19623064; 19.06.1996 DE 19624418
(43) Veröffentlichungstag der Anmeldung: 07.04.1999
(73) Patentinhaber: BAYER AG, 51368 Leverkusen (DE)
(72) Erfinder: STEFFAN, Guido, D-51519 Odenthal (DE); HARDENBICKER, Georg, D-51688 Wipperfürth (DE)
(86) Internationale Anmeldenummer: EP9702933
(87) Internationale Veröffentlichungsnummer: WO97047605

(56) Entgegenhaltungen:
- EP-A- 0 377 905
- CHEMICAL ABSTRACTS, vol. 106, no. 28, 1987 Columbus, Ohio, US; abstract no. 176415k, Seite 733; Spalte 2; XP002040387 & CN 85100015 (TIANJIN UNIVERSITY),10-11-85
- ANGEWANDTE CHEMIE INTERNATIONAL EDITION., Bd. 13, Nr. 3, 1974, WEINHEIM DE, Seite 210 XP002040386 G.BECK: "HIGHLY CHLORINATED SYSTEMS BY RING CLOSURE REACTIONS."

## Beschreibung

Die Erfindung betrifft ein Verfahren zur Herstellung von 4,5,6-Trichlor- und 2,4,5,6-Tetrachlorpyrimidin, ausgehend von 3-Dimethylaminopropionitril, sowie eine kristalline Zwischenproduktform.

Gemäß US-A 3 509 032 werden 4,5,6-Trichlor- und 2,4,5,6-Tetrachlorpyrimidin durch Chlorierung von 3-Dimethylaminopropionitril mit Cl₂ unter Bestrahlung von UV-Licht erhalten, wobei die Temperatur der Chlorierung zeitweise oberhalb von 150°C, insbesondere bei 180 bis 220°C, liegen muß, um die gewünschten Produkte entstehen zu lassen (siehe Spalte 2, Zeile 51 bis 53 und Beispiel 2 und 3). Derartig hohe Chlorierungstemperaturen sind jedoch bei einer technischen Realisierung der Verfahrensweise nachteilig.

Gemäß DE-A 3 900 917 wird 4,5,6-Trichlorpyrimidin ausgehend vom Hydrochlorid des 3-Dimethylaminopropionitrils durch eine einstufige Chlorierung bei einer Temperatur von 120 bis 130°C hergestellt. Diese Verfahrensweise ist aber noch in einigen Punkten verbesserungsfähig, insbesondere hinsichtlich ihrer Eignung für großtechnisch dimensionierte Ansätze.

Außerdem weisen die im Stand der Technik beschriebenen Verfahren zumeist den Nachteil auf, daß bei der anschließenden Destillation unlösliche Destillationsrückstände gebildet werden, die nur mit erheblichem Aufwand aus den Destillatoren zu entfernen sind. Zudem gasen die so erhaltenen Produkte sehr stark bei der Destillation.

Aufgabe der vorliegenden Erfindung war es daher, ein Verfahren zur Herstellung von 4,5,6-Trichlor- und 2,4,5,6-Tetrachlorpyrimidinen ausgehend von 3-Dimethylaminopropionitril bereitzustellen, das die genannten Nachteile nicht aufweist.

Es wurde ein Verfahren zur Herstellung von 4,5,6-Trichlor- und 2,4,5,6-Tetrachlorpyrimidin durch Umsetzung von 3-Dimethylaminopropionitril mit HCl und Cl₂ gefunden, bei dem man
a) in einem ersten Reaktionsschritt 3-Dimethylaminopropionitril in einem Lösungsmittel, das gegen HCl und Cl₂ inert ist, bei einer Temperatur von -10 bis 55°C mit HCl, vorzugsweise 1 bis 6 mol, und Cl₂, vorzugsweise 2 bis 4 mol, jeweils bezogen auf 1 mol 3-Dimethylaminopropionitril, umsetzt und
b) in einem zweiten Reaktionsschritt das Reaktionsgemisch des ersten Reaktionsschrittes bei einer Temperatur von über 55°C bis 120°C, vorzugsweise von 65 bis 120°C mit Cl₂, vorzugsweise 3 bis 5 mol, bezogen auf 1 mol eingesetztes 3-Dimethylaminopropionitril, gegebenenfalls in Gegenwart eines Katalysators, umsetzt,

das dadurch gekennzeichnet ist, daß der zweite Reaktionsschritt in Gegenwart des Reaktionsproduktes aus dem 1. Reaktionsschritt erfolgt, welches in mikrokristalliner Form mit einer mittleren Kristallgröße von ≤ 10 µm vorliegt.

Als gegen HCl und Cl₂ unter Reaktionsbedingungen inerte Lösungsmittel sind vorzugsweise solche zu verstehen, die unter den genannten Reaktionsbedingungen des erfindungsgemäßen Verfahrens inert sind. Es kommen beispielsweise chlorierte, aliphatische und aromatische Kohlenwasserstoffe, wie Chloroform, Tetrachlorkohlenstoff, Chlorbenzol, Dichlorbenzol, Isododecan, Phosphoroxychlorid oder Gemische davon in Frage. Besonders bevorzugt ist Phosphoroxychlorid (POCl₃).

Der erste Reaktionsschritt des erfindungsgemäßen Verfahrens wird bevorzugt bei einer Temperatur von 15 bis 25°C durchgeführt. Er kann beispielsweise batchweise oder kontinuierlich durchgeführt werden.

In der batchweisen Verfahrensvariante wird beispielsweise so vorgegangen, daß das 3-Dimethylaminopropionitril vorzugsweise in dem gegenüber Cl₂ und HCl inerten Lösungsmittel vorgelegt wird und HCl eingeleitet wird. Die Menge der einzuleitenden HCl beträgt im allgemeinen 1 bis 6 mol, vorzugsweise 3 bis 4 mol, bezogen auf 1 mol des eingesetzten 3-Dimethylaminopropionitrils. Das entstehende HCl-Addukt des Nitrils ist insbesondere dann im erfindungsgemäßen Temperaturbereich gut löslich, wenn als Lösungsmittel Phosphoroxychlorid verwendet wird. Die bei der HCl-Einleitung unter HCl-Addukt-Bildung entstehende Wärme wird vorzugsweise durch Kühlung abgeführt. Daran schließt sich dann die Umsetzung mit vorzugsweise 2 bis 4 mol Cl₂ pro 1 mol 3-Dimethylaminopropionitril an. Cl₂ wird dabei unter HCl-Entwicklung in die Reaktionsmischung der HCl-Umsetzung eingeleitet. Die Einleitung erfolgt vorzugsweise bei 15 bis 20°C.

Bevorzugt ist bei der oben beschriebenen batchweisen Variante 1 bis 2 mol HCl einzuleiten, bevor mit der Cl₂-Einleitung begonnen wird. Die HCl-Addukt-Bildung des Nitrils wird dann durch die bei der Chlorierung entstehende HCl komplettiert.

Die batchweise Verfahrensvarinate wird vorzugsweise für kleine Ansätze gewählt, insbesondere für Ansätze kleiner 10 mol, bezogen auf 3-Dimethylaminopropionitril.

Besonders bevorzugt ist es, die Umsetzung des ersten Reaktionsschrittes des erfindungsgemäßen Verfahrens kontinuierlich zu betreiben. Bei der kontinuierlichen Arbeitsweise werden vorzugsweise das gegen Cl₂ und HCI inerte Lösungsmittel, insbesondere Phosphoroxychlorid, 3-Dimethylaminopropionitril, HCl und Cl₂ kontinuierlich in einem Reaktor zusammengeführt. Die Menge an HCl beträgt vorzugsweise 1 bis 2 mol pro 1 mol 3-Dimethylaminopropionitril.

Die Menge an Cl₂ entspricht der eingangs erwähnten. Die bei der Chlorierung entstehende HCl verbleibt vorzugsweise in der Reaktionsmischung und braucht daher von außen nicht zugeführt zu werden. Die während der Reaktion freiwerdende Wärme wird vorzugsweise durch Kühlung abgeführt. Die Temperatur, für die kontinuierliche Verfahrensweise, wird besonders bevorzugt bei 15 bis 25°C gehalten.

Besonders bevorzugt erfolgt die Umsetzung des 1. Reaktionsschrittes voll kontinuierlich, worunter die kontinuierliche Zufuhr von Reaktanden und das kontinuierliche Abführen von Reaktionsprodukt verstanden wird.

Sofern Phosphoroxychlorid als bevorzugtes Lösungsmittel verwendet wird, liegt die kontinuierlich gebildete Reaktionsmischung des ersten Reaktionsschrittes bei einer Temperatur von -10 bis +55°C in Form einer Lösung vor.

Die bei dem ersten Reaktionsschritt des erfindungsgemäßen Verfahrens gebildeten Reaktionsprodukte sind vorzugsweise die Hydrochloride des chlorierten 3-Dimethylaminopropionitrils, die vorzugsweise den Formeln (I) und (II) entsprechen 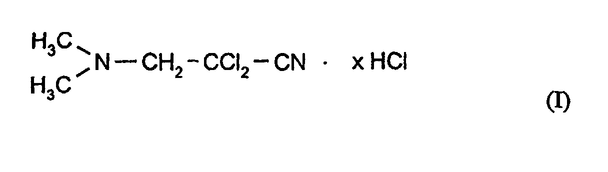 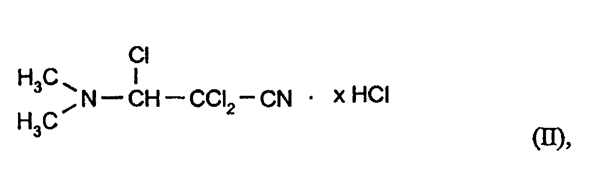 worin
- x: eine Zahl von 1 bis 3, vorzugsweise 3 bedeutet.

Besonders bevorzugt wird ein Produktgemisch gebildet, in dem die Verbindungen der Formeln (I) und (II) in einem Verhältnis von 10:1 bis 2:1 vorliegen.

Die Menge des verwendeten Lösungsmittels kann über weite Bereiche variiert werden. Bevorzugt ist es, 4 bis 10 Teile des Lösungsmittels pro Teil des eingesetzten 3-Dimethylaminopropionitrils einzusetzen.

In einer besonders bevorzugten Ausführungsform des erfindungsgemäßen Verfahrens erfolgt die Zusammenführung des Reaktionsgemisches aus dem ersten Reaktionsschritt und weiteren Cl₂ bei der Temperatur des zweiten Reaktionsschrittes semikontinuierlich, d.h. daß die Zusammenführung, vorzugsweise in einen zweiten Reaktor, solange erfolgt, wie die Dimension dieses Reaktors eine Befüllung erlaubt. Dabei kann gegebenenfalls mitverwendeter Katalysator beispielsweise mit dem zugeführten Reaktionsgemisch des ersten Reaktionsschrittes zugeführt werden oder beispielsweise bereits in dem Reaktor vorhanden sein.

Bei dem zweiten Reaktionsschritt wird HCl gebildet. Diese entsteht einerseits bei der Chlorierung und wird andererseits aus dem HCl-Addukt des ersten Reaktionsschrittes unter Bildung eines Monohydrochlorids freigesetzt.

Bei der Überführung der Reaktionsmischung des ersten Reaktionsschrittes in den zweiten Schritt, vorzugsweise in den zweiten Reaktor, kommt es zur kristallinen Ausfällung. Außerdem kommt es beim Übergang in den bei höherer Temperatur ablaufenden zweiten Reaktionsschritts (gegenüber dem ersten Reaktionsschritt) zur HCl-Entwicklung. Bei der Überführung der Reaktionsmischung in den 2. Reaktionsschritt entstehen dabei zunächst grobkristalline Ausfällungen. Nach kurzer Zeit sind dann feinere Kristalle (Mikrokristallisat) zu beobachten.

Sowohl bei den groben als auch bei den feinen Kristallen handelt es sich um Kristalle bzw. Mischkristalle der Monohydrochloride der Formeln (Ia) und/oder (IIa).

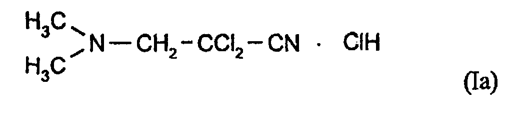

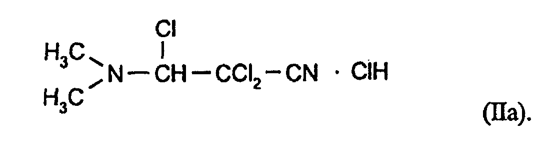

Diese fallen zunächst als relativ grobe Kristalle an, welche eine mittlere Kristallgröße von >10 µm besitzen. Bevorzugt fallen diese groben Kristalle in Form von farblosen Plättchen an, die quadratisch sind und eine Kantenlänge von 0,05 bis 0.2mm und eine Dicke von etwa 0,01mm besitzen. Bevorzugt enthalten diese Kristalle die Verbindungen Ia und IIa in einem Gewichtsverhältnis von 10:1 bis 2:1.

Die Erfindung betrifft auch Kristallisate mit einer mittleren Kristallgröße von ≤ 10 µm, enthaltend die Verbindungen der Formel Ia und/oder IIa.

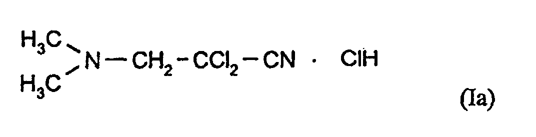

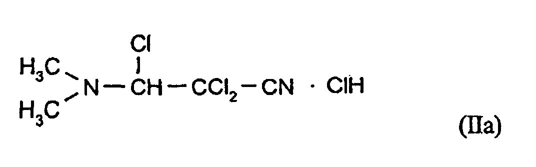

Bevorzugt enthalten diese die Verbindungen der Formeln Ia und IIa in einem Gewichtsverhältnis von 10:1 bis 2:1.

Bevorzugt liegt das Mikrokristallisat in Form von Nadeln bzw. Stäbchen vor, die eine Länge von 2 bis 6 µm und eine Dicke von 0,5 bis 1 µm besitzen.

Die Erfindung betrifft weiterhin ein Verfahren zur Herstellung dieses Mikrokristallisats, das dadurch gekennzeichnet ist, daß man 3-Dimethylaminopropionitril in einem gegen HCl und Cl₂ inerten Lösungsmittel bei einer Temperatur von -10 bis +55°C mit 1 bis 6 mol HCl und 2 bis 4 mol Cl₂, jeweils bezogen auf 1 mol 3-Dimethylaminopropionitril, umsetzt und anschließend die so erhaltenen Reaktionsmischung bei einer Temperatur von 65 bis 85°C tempert.

In einer besonders bevorzugten Ausführungsform des erfindungsgemäßen Verfahrens zur Herstellung der Chlorpyrimidine beträgt das Verhältnis von Mikrokristallsat zu einer groben Kristallform 95:5 bis 50:50.

Das erfindungsgemäße Verfahren eignet sich insbesondere für Ansätze > 1 kmol, bezogen auf 3-Dimethylaminopropionitril.

Der zweite Reaktioinsschritt schließt sich vorzugsweise unmittelbar an den 1. Reaktionsschritt an, so daß die Verweilzeit, die zwischen den beiden Schritten liegt, möglichst klein gehalten wird.

Isoliert man das feinkristalline Monohydrochlorid, so kann man es als Impfkristalle für den zweiten Reaktionsschritt verwenden. In dem Fall wird der gleiche vorteilhafte Effekt erzielt, auch ohne die Bildung der feinkristallinen Ausfällung abzuwarten.

Die Überführung des Reaktionsproduktes des 1. Reaktionsschrittes in den zweiten Reaktor erfolgt vorzugsweise so, daß der Anteil des sich nur langsam bildenden Mikrokristallisats gegenüber der groben Kristallform nicht allzu klein wird, da gefunden wurde, daß die grobe Kristallform unter den Reaktionsbedingungen nicht stabil ist. Bevorzugt beträgt das Verhältnis von Mikrokristallisat zur groben Kristallform 95:5 bis 50:50.

In einer ganz besonders bevorzugten Ausführungsform des zweiten Reaktionsschrittes erfolgt die Umsetzung in Gegenwart eines Katalysators. Als mögliche Katalysatoren sind beispielsweise organische Katalysatoren zu nennen. Geeignete organische Katalysatoren sind beispielsweise offenkettige oder cyclische Carbonamide, wie C₁-C ₁₂-Dialkylformamide, insbesondere Dimethylformamid, Dibutylformamid, Methyldodecylformamid, N-C₁-C₁₂-Alkylpyrrolidone, wie N-Methylpyrrolidon-(2), N-C₁-C₁₂-Alkylcaprolactame, wie N-Methylcaprolactam, ferner Trialkylphosphite, Triarylphosphite, Triarylphosphinoxide, wobei Aryl vorzugsweise gegebenenfalls substituiertes Phenyl bedeutet. Besonders bevorzugt ist Triphenylphosphinoxid.

Die Katalysatoren können auch im Gemisch untereinander eingesetzt werden, vorzugsweise in Mengen von 1 bis 10, vorzugsweise 2 bis 5 Gew.-%, bezogen auf eingesetztes 3-Dimethylaminopropionitril.

Ganz besonders bevorzugt erfolgt der zweite Reaktionsschritt des erfindungsgemäßen Verfahrens in Gegenwart von Radikalabfängen. Bevorzugt ist Sauerstoff, insbesondere in Form von Luft. Luft wird beispielsweise bevorzugt in einer Menge von 10 bis 100 1 pro 1 kg eingesetztes 3-Dimethylaminopropionitril, insbesondere kontinuierlich über die Reaktionszeit, in die Reaktionsmischung zugeführt.

Ganz besonders bevorzugt ist das erfindungsgemäße Verfahren, bei dem die im zweiten Reaktionsschritt gebildete HCI, gegebenenfalls zusammen mit überschüssigem Cl₂ des zweiten Reaktionsschrittes in den ersten Reaktionsschritt rückgeführt wird, wodurch von außen zuzuführende HCl nicht weiter benötigt wird. Das erfindungsgemäße Verfahren in einer besonders bevorzugten Ausführungsform ist daher bezüglich HCl autark.

Bei dem erfindungsgemäßen Verfahren entstehen vorzugsweise 4,5,6-Trichlor- und 2,4,5,6-Tetrachlorpyrimidin im Gemisch. Die Zusammensetzung zugunsten eines der beiden Pyrimidine läßt sich vorzugsweise über die Temperatur steuern. Dabei wird das Trichlorpyrimidin beispielsweise bei gleicher Reaktionszeit bevorzugt bei einer Reaktionstemperatur von 85 bis 105°C gebildet, wohingegeben das 2,4,5,6-Tetrachlorpyrimidin vorzugsweise bei einer Temperatur von 65 bis 75°C entsteht. So erhaltene Chlorpyrimidinmischungen weisen vorzugsweise ein Verhältnis von Trichlor- zu Tetrachlorpyrimidin von 4:1 bis 1:30 auf.

Das entstehende 4,5,6-Trichlor- und 2,4,5,6-Tetrachlorpyrimidin sowie das verwendete Lösungsmittel werden zwecks Isolierung bzw. Rückgewinnung vorzugsweise fraktioniert destilliert Nach Abdestillieren des jeweiligen Produkts kann aus dem Destillationsrückstand in Gegenwart von wenig Aktivkohle noch jeweils eine zweite Fraktion des Produkts erhalten werden. Die verbleibenden relativ geringen Destillationsrückstände sind gut z.B. in o-Dichlorbenzol löslich und können in dieser Form beispielsweise verbrannt werden.

4,5,6-Trichlor- und 2,4,5,6-Tetrachlorpyrimidin sind beispielsweise wichtige Vorprodukte für die Herstellung fluorhaltiger Reaktivkomponenten, wie sie in Reaktivfarbstoffen eingebaut werden (s. DE-A 1 644 203) und eignen sich ebenfalls als Vorprodukte für Pflanzenschutzmittel sowie Pharmazeutika.

### Beispiele

### Beispiel 1

### Herstellung von 4,5,6-Trichlorpyrimidin

In zwei hintereinander geschalteten Reaktoren 1 und 2 wurden jeweils 85 g (50 ml) Phosphoroxychlorid vorgelegt. In den hinteren der beiden Reaktoren (Reaktor 2) wurden zudem noch 5 g Triphenylphosphinoxid gegeben. Die Umsetzung erfolgt wasserfrei.

Bei einer durch Kühlung bei 15 bis 20°C gehaltenen Temperatur wurden im Laufe von 13 Stunden in den Reaktor 1 gleichmäßig eindosiert:
1. Eine Lösung von 600 g (6,12 mol) an 3-Dimethylaminopropionitril in 3.340 g Phosphoroxychlorid,
2. ca. 500 g (17,7 mol) Chlorwasserstoff sowie
3. ca. 850 g (12 mol) Chlor,
wobei die Chlorwasserstoff- und Chlor-Dosierraten anfangs etwas höher gewählt wurden und nach Inbetriebnahme des 2. Reaktors etwas zurückgenommen wurden, als das Abgas aus Reaktor 2 im Reaktor 1 mit benutzt wurde.

Die Menge des Reaktionsgemisches im Reaktor 1 wurde bei 50 bis max. 100 ml durch Abpumpen oder Überlauf in den Reaktor 2 gehalten.

Sobald das Reaktionsgemisch als klare Lösung aus Reaktor 1 in Reaktor 2 überlief, wurde mit 200 bis 300 mg des gemäß Beispiel 4 erhaltenen Mikrokristallisats angeimpft und anschließend mit der Eindosierung von 2.300 g (32,4 mol) Chlor im Laufe von 13 Stunden und von trockener Luft (ca. 0,5 ml pro Sekunde) in den Reaktor 2 begonnen, wobei die Eindosierung in den Reaktor 2 zunächst bei einer Temperatur von ca. 85°C erfolgte. Nachdem Abgas aus dem Reaktor 2 zu entweichen begann, wurde ein Strahlwäscher über Reaktor 1 in Betrieb genommen, um dort das Abgas aus Reaktor 2 zu nutzen.

Die Temperatur im Reaktor 2 wurde dann zunächst auf 95 bis 97°C und nach einer weiteren Stunde auf 102 bis 104°C erhöht und dort 13 Stunden lang gehalten. Nach 5 und 13 Stunden wurden jeweils weitere 5 g Triphenylphosphinoxid in den Reaktor 2 eindosiert.

Nachdem der Reaktor 2 (Fassungsvermögen = 41) nahezu gefüllt war, wurde nach Stoppen aller Produktzufuhren in den Reaktor 1 letzterer völlig in den Reaktor 2 entleert.

Im Laufe von weiteren 12 Stunden wurden dann ca. 1.100 g (15,5 mol) Chlor (und weiterhin die oben genannte Menge Luft) in den Reaktor 2 mit allmählich abnehmender Dosiergeschwindigkeit so eingeleitet, daß anfangs das Abgas nahezu farblos und später nur schwach grün war.

Das Reaktionsende ist erreicht, sobald sich die milchig werdende Reaktionsmischung im Reaktor 2 allmählich in eine klare helle Lösung umwandelt. Die Aufarbeitung schloß sich sodann daran an.

### Aufarbeitung

Aus der so erhaltenen Lösung wurde über eine Kolonne zunächst Phosphoroxychlorid, das wiederverwendet wurde, vollständig abdestilliert. Man erhielt ca. 3.350 g Phosphoroxychlorid. Danach wurden im Vakuum 1.048 g eines Gemisches aus Triund Tetrachlorpyrimidin abdestilliert, wobei bis zu einer maximalen Sumpftemperatur von 180°C bei 12 mbar erhitzt wurde.

Der Destillationsrückstand wurde nach Zusatz von 5 g trockener Aktivkohle 1 Stunde bei 195°C unter geringer Chlorabspaltung betempert. Danach wurden bei 10 bis 12 mbar und bis zu einer maximalen Sumpftemperatur von 200°C weitere 54 g Produktgemisch erhalten. Der Rückstand (81 g, davon 15 g Triphenylphosphinoxid-Derivat und 5 g Aktivkohle) wurde mit 50 g 1,2-Dichlorbenzol zu einer dünnflüssigen Suspension verrührt.

Zusammen mit ca. 1 % Produktgemisch im Phosphoroxychlorid-Destillat wurden insgesamt (nach GC-Analyse) 756 g 4,5,6-Trichlorpyrimidin (= 68 % der Theorie, bezogen auf eingesetztes 3-Dimethylaminopropionitril) und 348 g Tetrachlorpyrimidin (= 26,5 % der Theorie, bezogen auf eingesetztes 3-Dimethylaminopropionitril) erhalten, die durch Rektifikation getrennt wurden. Dies entspricht einer Gesamtausbeute von 94,5 % der Theorie, bei Einsatz von 99 %igen 3-Dimethylaminopropionitril.

Gleiche Ergebnisse wurden auch bei 10 bis 12 mol sowie 1 bis 2 kmol Ansätzen erhalten, während bei einer Verfahrensführung, wie sie aus DE-A 3 900 917 bekannt ist die Ausbeute von 95 bis 90 % bei 0,5 bis 2 Mol-Ansätzen, auf 75 bis 70 % bzw. bei 10 bis 12 Mol-Ansätzen und auf 65 bis 60 % bei 1-2 kmol-Ansätzen absanken, jeweils bezogen auf eingesetztes 3-Dimethylaminopropionitril

### Beispiel 2

### Herstellung von 2,4,5,6-Tetrachlorpyrimidin

In zwei hintereinander geschalteten Reaktoren 1 und 2 (wie in Beispiel 1) wurden 85 g (50 ml) Phosphoroxychlorid im Reaktor 1 und 125 g (75 ml) Phosphoroxychlorid sowie 5 g Triphenylphosphinoxid in den hinteren Reaktor (Reaktor 2) vorgelegt.

Bei einer durch Kühlung bei 15 bis 20°C gehaltenen Temperatur wurden im Laufe von 16 3/4 Stunden in den Reaktor 1 gleichzeitig eindosiert:
1. Eine Lösung von 600 g (6,12 mol) an 3-Dimethylaminopropionitril in 3.300 g Phosphoroxychlorid,
2. ca. 600 g (19,5 mol) Chlorwasserstoff sowie
3. ca. 890 g (12,53 mol) Chlor, i
wobei die Chlorwasserstoff- und Chlor-Dosieiraten anfangs etwas höher eingestellt wurden (ca. 40 g HCl/h bzw. ca. 55 bis 60 g Chlor/h) und nach Start von Reaktor 2 etwas reduziert wurden, als das Abgas aus Reaktor 2 im Reaktor 1 mit genutzt wurde.

Die Menge des Reaktionsgemisches im Reaktor 1 wurde bei 50 bis max. 100ml gehalten, indem sie kontinuierlich in Reaktor 2 durch Überlauf abgelassen wurde.

Sobald die Reaktionsmischung als Lösung aus Reaktor 1 im Reaktor 2 übertrat, wurde bei einer Temperatur von ca. 75°C mit der Einleitung von 3.600 g (50,7 mol) Chlor im Laufe von 16 3/4 Stunden und von trockener Luft (ca. 0,5 ml pro Sekunde) begonnen.

Es wurden kurz darauf 200 bis 300 mg Impfkristalle der feinkristallinen Monohydrochloridmischung von 2,2-Dichlor- und 2,2,3-Trichlor-3-dimethylaminopropionitril im Reaktor 2 zugesetzt.

Sobald das Abgas aus Reaktor 2 zu entweichen begann, wurde ein Strahlwäscher über Reaktor 1 in Betrieb genommen, um dort das Abgas aus Reaktor 2 zu nutzen. Die Temperatur in Reaktor 2 wurde während der gesamten 16 3/4 Stunden bei 74 bis 76°C gehalten. Nach 7 und 14 Stunden der 16 3/4 Stunden wurden jeweils weitere 5 g Triphenylphosphinoxid im Reaktor 2 eindosiert.

Sobald Reaktor 2 (Fassungsvermögen =41) nach Ablauf von 16 3/4 Stunden nahezu gefüllt war, wurden alle Eindosierungen im Reaktor 1 gestoppt und der Inhalt des letzteren in den Reaktor 2 überführt.

Im Laufe von weiteren 22,5 Stunden wurden dann 820 g (11,55 mol) Chlor und weiterhin die oben angegebene Menge Luft in den Reaktor 2 eingeleitet mit allmählich abnehmender Dosiergeschwindigkeit, so daß das Abgas stets nur schwach grün gefärbt war. Nach 8 Stunden der 22,5 Stunden wurden noch einmal 5 g Triphenylphosphinoxid zugesetzt. Die Temperatur wurde hierbei während der ersten 14 Stunden bei 74 bis 76°C gehalten, und dann in 8 weiteren Stunden allmählich von 80 auf 90°C gesteigert und in der letzten halben Stunde, nachdem sich die milchig wirkende Reaktionsmischung in Reaktor 2 allmählich in eine klare helle Lösung umgewandelt hatte, auf 95°C gehalten.

Aus der so erhaltenen Lösung wurde über eine Kolonne zunächst das Phosphoroxychlorid vollständig abdestilliert. Man erhielt ca. 3.410 g Phosphoroxychlorid. Danach wurde im Vakuum 1.105 g eines Gemischs aus Tri- und Tetrachlorpyrimidin abdestilliert, wobei bis zu einer maximalen Sumpftemperatur von 180°C bei 12 mbar erhitzt wurde.

Der Destillationsrückstand wurde nach Zusatz von 5 g trockener Aktivkohle eine Stunde bei 195°C unter geringer Chlorabspaltung getempert. Danach wurden bei 10 bis 12 mbar und bis zu einer maximalen Sumpftemperatur von 205°C weitere 122 g Produktgemisch erhalten.

Der Rückstand (105 g, davon ca. 20 g Triphenylphosphinoxid-Derivate und 5 g Aktivkohle) war bei 130°C noch flüssig und wurde mit 100 g 1,2-Dichlorbenzol zu einer auch bei Raumtemperatur noch dünnflüssigen Suspension verrührt.

Zusammen mit ca. 0,5 % Produkt des Produktgemisch im Phosphoroxychlorid-Destillat wurden so insgesamt (nach GC-Analyse) 102 g 4,5,6-Trichlorpyrimidin (= 9,2% der Theorie, bezogen auf 3-Dimethylaminopropionitril) und 1.139g Tetrachlorpyrimidin (= 86,2 % der Theorie, bezogen auf 3-Dimethylaminopropionitril) erhalten, die durch Rektifikation getrennt wurden. Dies entspricht einer Gesamtausbeute von 95,4 % der Theorie, bei Einsatz von 99 %igem Dimethylaminopropionitril. Gleiche Ausbeuten wurden auch in 10 und 12 mol-Ansätzen erhalten.

### Beispiel 3

Es wurde in gleicher Weise wie in Beispiel 2 verfahren, jedoch wurde die Zeit der kontinuierlichen Chlorierung von 16 3/4 Stunden auf 19 Stunden verlängert, wobei die Temperatur im Reaktor 2 bei 70°C gehalten wurde, und danach 19 Stunden bei 70°C, 6 Stunden bei 75 bis 80°C, 4 Stunden bei 80 bis 85°C und 1 Stunde bei 90°C chloriert. Es wurde eine Ausbeute von 93 % der Theorie, bezogen auf eingesetztes 3-Dimethylaminopropionitril an Tetrachlorpyrimidin und 3 % der Theorie an 4,5,6-Trichlorpyrimidin erhalten.

### Beispiel 4

Herstellung und Charakterisierung der grob- und mikrokristallinen Formen von 2,2-Dichlor-3-dimethylamino-propionitril-monohydrochlorid sowie der mikrokristallinen Form von 2,2,3-Trichlor-3-dimethylamino-propionitril-monohydrochlorid
a) In eine Mischung von 580 g Phosphoroxichlorid und 100 g 3-Dimethylaminopropionitril (ca. 98 %ig) wurden zuerst bei 15 bis 25°C 100 g HCl-Gas in ca. 45 Minuten unter Kühlung eingeleitet und dann in die erhaltene klare Lösung 142 g Chlor unter starker Kühlung bei 10 bis 15°C in ca. 30 Minuten. Die so erhaltene klare Lösung wurde schnell (in ca. 10 Minuten) auf 50°C erwärmt, wobei noch sehr wenig Chlor (ca. 5 g) eingeleitet wird und viel HCl ausgast. Sehr bald schieden sich grobe klare Kristalle ab.
b) Die Hälfte der in a) erhaltenen Reaktiionsmischung wurde über eine Glassinternutsche filtriert, das erhaltene Kristallisat wurde mit Toluol und Petrolether gewaschen, um es von Phosphoroxichlorid zu befreien und im Vakkum bei 50°C 2 Stdn. getrocknet. Es wurden 76 g farbloser Plättchen erhalten, die durch Elementaranalyse, Massen- und NMR-Spektrum als Monohydrochlorid des 2,2-Dichlor-3-dimethylamin-propionitril identifiziert wurden. Es handelte sich um quadratische Plättchen von 0,05 bis 0,2 mm Kantenlänge und 0,01 mm Dicke.
c) Die zweite Hälfte der o.g. Reaktionsmischung wurde auf 75°C erwärmt Nach ca. 10 Minuten wandelten sich die o.g. groben Plättchen um zu einer sehr feinen Kristallmasse, wodurch die Reaktionsmischung sehr dickflüssig wurde. Nach 10 bis 15 Minuten weiteren Temperns zur Vervollständigung der Umwandlung wurde das Kristallisat wie oben filtriert, gewaschen und getrocknet. Es wurden 74g farbloser Mikrokristalle erhalten, die durch Elementaranalyse, Gaschromatographie, Massen- und NMR-Spektrum identifiziert werden als Mischung aus
   ca. 90 Mol-Prozent des o.g. Monohydrochlorids von 2,2-Dichlor-3-dimethylaminopropionitril und
   ca. 10 Mol-Prozent des Monohydrochlorids von 2,2,3-Trichlor-3-dimethylaminopropionitril.
   Die mikroskopische Untersuchung zeigte, daß das Mikrokristallisat in Form von Nadeln bzw. Stäbchen mit einer Länge von 2 bis 6 Mikrometer und ca. 0,5 bis 1 Mikrometer Stärke anfällt, die weitgehend agglomeriert waren.
d) Ein weiterer Ansatz wurde ebenso durchgeführt, wie unter a) beschrieben, jedoch werden beim Erwärmen auf 50°C statt 5 g weitere 35 g Chlor eingeleitet.

Danach wurde - wie unter c) beschrieben - auf 75°C erwärmt und weiterhin Chlor eingeleitet (insgesamt ca. weitere 40 g).

Nach ca. 15 Minuten wandelten sich die groben Plättchen, die beim Erwärmen auf 50°C auskristallisierten, in das feine Kristallisat um, das nach 15 Minuten weiteren Temperns, wie beschrieben, isoliert wurde.

Die oben durchgeführten Untersuchungen zeigten, daß das Produkt nahezu reines Monohydrochlorid des 2,2,3-Trichlor-3-dimethylaminopropionitrils ist. Es zeigte bei der mikroskopischen Untersuchung den gleichen kristallhabitus wie das o.g. entsprechende Gemisch aus Dichlor- und Trichlorderivat.

### Beispiel 5 (Tetrachlorpyrimidin im 1.000 l-Reaktor)

In einem 1.000 l-Stahlemail-Reaktor (mit Gaseinleitung durch das Bodenventil wurden 165 kg Phosphoroxichlorid (1001) und 1,5 kg Triphenylphospinoxid vorgelegt und auf 75°C erwärmt. In einem darüberliegenden 25-lGlasreaktor (mit Intensiv-Sole-Kühlung) wurden 16 kg Phosphoroxichlorid (ca. 10l) vorgelegt.

Dann wurden im Laufe von 17 Stdn. gleichmäßig und simultan in den 25 1-Glasreaktor eindosiert:
980 kg Phosphoroxichlorid
180 kg 3-dimethylaminopropionitril
120 kg Chlorwasserstoff und
274 kg Chlor
wobei die Temperatur unter intensivem Durchmischen und Kühlen bei 10 bis 15°C gehalten wurde.

Das Reaktionsvolumen im 25 l-Glasreaktor wurde durch Überlauf in den 1.000l Stahlemail-Reaktor bei 201 gehalten.

Sobald Produkt aus dem 251- in den 1.000l-Reaktor überlief wurden dort ca. 50 g Impfkristalle (Mikrokristallisat aus 2,2-Dichlor- und 2,2,3-Trichlor-3-dimethylaminopropionitril-monohydrochlorid/Herstellung siehe Beispiel 4) zugesetzt

Dann wurden im Laufe von insgesamt 17 Stunden bei 75°C gleichmäßig insgesamt 762 kg Chlor (und pro Stunde ca. 100 l trockene Luft) durch das Bodenventil in den 1.000 l-Reaktor eindosiert. Während dieser 17 Stdn. wurden nach 7 und 13 Stdn. jeweils je 1,5 kg Triphenylphosphinoxid in den 1.000l-Reaktor eingegeben.

Nach Ablauf dieser 17 Stdn. wurde der Inhalt des Glasreaktors in den 1.000l-reaktor abgelassen.

Im Laufe von weiteren 21 Stdn. wurden dann insgesamt 166 kg Chlor (und weiterhin die o.g. Luftmenge) so in den 1.000 l-Reaktor eindosiert, daß das Abgas stets schwach grün gefärbt war. Dabei wurden nach Ablauf von 9 dieser 21 Stdn. noch einmal 1,5 kg Triphenylphosphinoxid zugegeben.

Die Temperatur im 1.000l-Reaktor wurde während der ersten 14 Stdn. dieser 21 Stdn. bei 74 bis 76°C gehalten, dann in 6 Stdn. langsam auf 88°C gesteigert. Nach vollständiger Auflösung des Kristallisats wurde die Temperatur auf 95°C gesteigert und dort noch 1/2 Std. gehalten.

Aus der so erhaltenen Lösung wurde über eine 5-boden-Kolonne bei einem Rücklaufverhältnis von 1:1 zuerst das Phosphoroxichlorid (bei einem allmählich bis auf 60 mbar reduzierten Druck) praktisch vollständig abdestilliert. Dann wurden bei 10 bis 12 mbar bis zu einer Sumpftemperatur von 183°C das Gemisch aus Tri- und Tetrachlorpyrimidin (326 kg) abdestilliert.

Der Destillationsrückstand wurde nach Zusatz von 2 kg trockener Aktivkohle 2 Std. bei 195 bis 200°C getempert (mäßige Chlorabspaltung). Danach werden bei 9 bis 12 mbar und bis zu einer maximalen Sumpftemperatur von 212°C weitere 44,5 kg Produktgemisch durch Destillation erhalten.

Nach Gaschromatogramm enthielten die 370,5 kg Produktgemisch
5,6 Gew.% 4,5,6-Trichlorpyrimidin und
94,3 Gew.-% Tetrachlorpyrimidin, was einer Gesamtausbeute von 94,3 % der Theorie entspricht.

Der Destillationsrückstand ließ sich nach Zusatz von 40 kg 1,2-Dichlorbenzol vollständig aus dem Destillator entfernen (insgesamt 73 kg, davon 40 kg 1,2-Dichlorbenzol).

### Beispiel 6 (4,5,6-Trichlorpyrimidin in 1.000 l-Reaktor)

In derselben Apparatur, wie im Beispiel 5 beschrieben, wurden in den 1.000l-Reaktor 165 kg Phosphoroxichlorid (100l) und 1,5 kg-Triphenylphosphinoxid (Mischung auf 85°C erwärmt) und im 25 l-Reaktor 16 kg Phosphoroxichlorid (10 l) vorgelegt.

Dann wurden im Laufe von 14 Stunden gleichmäßig und simultan in den 25 l-Reaktor bei 10 bis 15°C:
995 kg Phosphoroxichlorid
180 kg 3-Dimethylamino-propionitril
115 kg Chlorwasserstoff und
266 kg Chlor
eindosiert.

Das Reaktionsvolumen im 25 l-reaktor wurde durch Überlauf in den 1:0001-Reaktor bei 20l gehalten.

Sobald Produkt aus dem 25 l-Reaktor in den 1.000 l-Reaktor überlief, wurden dort ca. 100 g Impfkristalle (Mikrokristallisat-Gemisch aus 2,2-Dichlor- und 2,2,3-Trichlor-3-dimethylaminopropionitril-monohydrochlorid/Herstellung s. Beispiel 4) zugesetzt.

Dann wurden im Laufe von insgesamt 14 Stdn. gleichmäßig insgesamt 675 kg Chlor (und pro Stunde ca. 1001 trockene Luft) durch das Bodenventil in den 1.000 l-Reaktor eindosiert, wobei die Anfangstemperatur von 85°C schnell auf 95 bis 97°C erhöht wurde und nach einer weiteren Stunde auf 103 bis 105°C und dort bis zum Ende der 14 Stunden gehalten wurde. Während dieser 14 Stdn. wurden jeweils 1,5 kg Triphenylphosphinoxid nach 6 und 13 Stdn. zusätzlich in den Reaktor gegeben). Nach Ablauf dieser 14 Stdn. wurde der Inhalt des 25 1-Reaktors in den 1.000 l-Reaktor abgelassen.

Im Laufe dieser Zeit wurde aus der Supension des Mikrokristallisats allmählich eine klare helle Lösung.

Diese Lösung wurde dann so aufgearbeitet, wie im Beispiel 5 beschrieben.

Man hielt insgesamt:
328 kg Produkt-Destillat, das nach Gaschromatogramm
72,2 Gew.-% 4,5,6-Trichlorpyrimidin und
27,6 Gew.-% Tetrachlorpyrimidin
enthält, was einer Gesamtausbeute von 93,8 % der Theorie entspricht.

Der Destillationsrückstand ließ sich nach Zusatz von 40kg 1,2-dichlorbenzol vollständig aus dem Destillator entfernen (insgesamt 67 kg, davon 40 kg 1,2-Dichlorbenzol).

## Patentansprüche

1. Verfahren zur Herstellung von 4,5,6-Trichlor- und 2,4,5,6-Tetrachlorpyrimidin durch Umsetzung von 3-Dimethylaminopropionitril mit HCl und Cl₂, indem man
a) in einem ersten Reaktionsschritt 3-Dimethylaminopropionitril in einem Lösungsmittel bei einer Temperatur von -10 bis 55°C mit HCI und Cl₂ umsetzt, wobei das Lösungsmittel gegen HCl und Cl₂ inert ist, und
b) in einem zweiten Reaktionsschritt das Reaktionsgemisch des ersten Reaktionsschrittes bei einer Temperatur von über 55°C bis 120°C mit Cl₂, gegebenenfalls in Gegenwart eines Katalysators, umsetzt,
**dadurch gekennzeichnet, daß** der zweite Reaktionsschritt in Gegenwart des Reaktionsproduktes aus dem 1 Reaktionsschritt erfolgt, welches in mikrokristalliner Form mit einer mittleren Kristallgröße von ≤ 10 µm vorliegt.

2. Verfahren gemäß Anspruch 1, **dadurch gekennzeichnet, daß** als Lösungsmittel Phosphoroxychlorid verwendet wird.

3. Verfahren gemäß Anspruch 1, **dadurch gekennzeichnet, daß** im ersten Reaktionsschritt die Umsetzung mit 1 bis 6 mol HCl und 2 bis 4 mol Cl₂, jeweils bezogen auf 1 mol 3-Dimethylaminopropionitril, erfolgt.

4. Verfahren gemäß Anspruch 1, **dadurch gekennzeichnet, daß** der zweite Reaktionsschritt b) bei einer Temperatur von 65 bis 120°C erfolgt.

5. Verfahren gemäß Anspruch 1, **dadurch gekennzeichnet, daß** die Umsetzung im zweiten Reaktionsschritt b) mit 3 bis 5 mol Cl₂, bezogen auf 1 mol eingesetztes 3-Dimethylaminopropionitril, erfolgt.

6. Verfahren gemäß Anspruch 1, **dadurch gekennzeichnet, daß** der erste Reaktionsschritt voll kontinuierlich betrieben wird.

7. Verfahren gemäß Anspruch 1, **dadurch gekennzeichnet, daß** die im zweiten Reaktionsschritt entstehende HCl in den ersten Reaktionsschritt rückgeführt wird.

8. Verfahren gemäß Anspruch 1, **dadurch gekennzeichnet, daß** im zweiten Reaktionsschritt in Gegenwart eines Katalysators aus der Reihe der offenkettigen oder cyclischen Carbonamide, Trialkylphosphite, Triarylphosphite oder Triphenylphosphinoxide, insbesondere Triphenylphosphinoxid oder einem Gemisch dieser Katalysatoren, gearbeitet wird.

9. Verfahren gemäß Anspruch 1, **dadurch gekennzeichnet, daß** die Umsetzung des zweiten Reaktionsschritts in Gegenwart von Radikalfängern erfolgt.

10. Verfahren gemäß Anspruch 1, **dadurch gekennzeichnet, daß** die Umsetzung des 2. Reaktionsschrittes semikontinuierlich erfolgt.

11. Verfahren gemäß Anspruch 1, **dadurch gekennzeichnet, daß** die Umsetzung von 3-Dimethylaminopropionitril mit Cl₂ im 1. Reaktionsschritt so erfolgt, daß 3-Dimethylaminopropionitril in Form seines HCl-Addukts gelöst vorliegt.

12. Kristallisat mit einer mittleren Kristallgröße von ≤ 10µm, enthaltend die Verbindungen der Formel Ia und/oder IIa

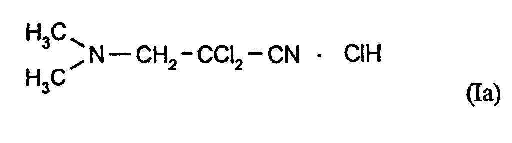

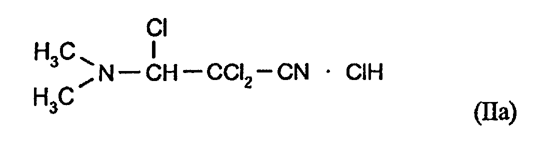

13. Verfahren zur Herstellung des Kristallisats gemäß Anspruch 12, **dadurch gekennzeichnet, daß** man 3-Dimethylaminopropionitril in einem gegen HClund Cl₂- inerten Lösungsmittel bei einer Temperatur von-10 bis 55°C mit 1 bis 6 mol HCl und 2 bis 4 mol Cl₂, jeweils bezogen auf 1 mol 3-Dimethylaminopropionitril, umsetzt und anschließend die so erhaltenen Reaktionsmischung bei einer Temperatur von 65 bis 85°C tempert.

## Claims

1. Process for the preparation of 4,5,6-trichloro-and 2,4,5,6-tetrachloropyrimidine by reacting 3-dimethylaminopropionitrile with HCl and Cl₂, which comprises
a) in a first reaction step reacting 3-dimethylaminopropionitrile in a solvent with HCl and Cl₂ at a temperature of from -10 to 55°C, the solvent being inert towards HCl and Cl₂, and
b) in a second reaction step reacting the reaction mixture from the first reaction step with Cl₂ at a temperature above 55°C to 120°C, optionally in the presence of a catalyst,
**characterized in that** the second reaction step is carried out in the presence of the reaction product from the 1st reaction step, which is in microcrystalline form with an average crystal size of ≤ 10 µm.

2. Process according to Claim 1, **characterized in that** the solvent used is phosphorus oxychloride.

3. Process according to Claim 1, **characterized in that** in the first reaction step the reaction is carried out using from 1 to 6 mol of HCl and from 2 to 4 mol of Cl₂, in each case based on 1 mol of 3-dimethylaminopropionitrile.

4. Process according to Claim 1, **characterized in that** the second reaction step b) is carried out at a temperature of from 65 to 120°C.

5. Process according to Claim 1, **characterized in that** the reaction in the second reaction step b) is carried out using from 3 to 5 mol of Cl₂, based on 1 mol of 3-dimethylaminopropionitrile used.

6. Process according to Claim 1, **characterized in that** the first reaction step is operated completely continuously.

7. Process according to Claim 1, **characterized in that** the HCl formed in the second reaction step is returned to the first reaction step.

8. Process according to Claim 1, **characterized in that** the second reaction step is carried out in the presence of a catalyst from the series consisting of open-chain or cyclic carboxamides, trialkyl phosphites, triaryl phosphites or triphenylphosphine oxides, in particular triphenylphosphine oxide or a mixture of these catalysts.

9. Process according to Claim 1, **characterized in that** the reaction in the second reaction step is carried out in the presence of free-radical scavengers.

10. Process according to Claim 1, **characterized in that** the reaction in the 2nd reaction step is carried out semi-continuously.

11. Process according to Claim 1, **characterized in that** the reaction of 3-dimethylaminopropionitrile with Cl₂ in the 1st reaction step is carried out such that 3-dimethylaminopropionitrile is in dissolved form in the form of its HCl adduct.

12. Crystals with an average crystal size of ≤ 10 µm comprising the compounds of the formula Ia and/or IIa

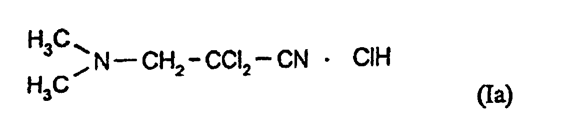

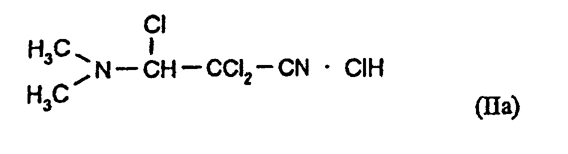

13. Process for the preparation of the crystals according to Claim 12, **characterized in that** 3-dimethylaminopropionitrile in a solvent which is inert towards HCl and Cl₂ is reacted with from 1 to 6 mol of HCl and from 2 to 4 mol of Cl₂, in each case based on 1 mol of 3-dimethylaminopropionitrile, at a temperature of from -10 to 55°C, and then the resulting reaction mixture is heat-treated at a temperature of from 65 to 85°C.

## Revendications

1. Procédé pour la préparation de 4,5,6-trichloro- et 2,4,5,6-tétrachloropyrimidine par réaction de 3-diméthylaminopropionitrile avec HCI et Cl₂, **caractérisé en ce que**
a) on fait réagir dans une première étape de réaction du 3-diméthylaminopropionitrile dans un solvant à une température de -10 à 55°C avec HCI et Cl₂, le solvant étant inerte par rapport à HCI et Cl₂, et
b) on fait réagir dans une seconde étape de réaction le mélange réactionnel de la première étape de réaction à une température de plus de 55°C à 120°C avec Cl₂, éventuellement en présence d'un catalyseur,
**caractérisé en ce que** la seconde étape de réaction est réalisée en présence du produit de réaction de la première étape de réaction, lequel est présent dans une forme microcristalline avec une taille moyenne de cristaux ≤ 10 µm.

2. Procédé selon la revendication 1, **caractérisé en ce que** l'on utilise comme solvant de l'oxychlorure de phosphore.

3. Procédé selon la revendication 1, **caractérisé en ce que** l'on réalise la réaction dans la première étape de réaction avec de 1 à 6 mol de HCI et de 2 à 4 mol de Cl₂, rapporté à chaque fois à 1 mol de 3-diméthylaminopropionitrile.

4. Procédé selon la revendication 1, **caractérisé en ce que** la seconde étape de réaction b) est réalisée à une température de 65 à 120°C.

5. Procédé selon la revendication 1, **caractérisé en ce que** la réaction est réalisée dans la seconde étape de réaction b) avec de 3 à 5 mol de Cl₂, rapporté à 1 mol de 3-diméthylaminopropionitrile utilisé.

6. Procédé selon la revendication 1, **caractérisé en ce que** l'on réalise la première étape de réaction de manière totalement continue.

7. Procédé selon la revendication 1, **caractérisé en ce que** l'on renvoie dans la première étape de réaction le HCI produit dans la seconde étape de réaction.

8. Procédé selon la revendication 1, **caractérisé en ce que** l'on travaille dans la seconde étape de réaction en présence d'un catalyseur choisi parmi des carbonamides à chaîne ouverte ou cycliques, des phosphites de trialkyle, des phosphites de triaryle ou des oxydes de triphénylphosphine, en particulier un oxyde de triphénylphosphine ou un mélange de ces catalyseurs.

9. Procédé selon la revendication 1, **caractérisé en ce que** l'on réalise la réaction de la seconde étape de réaction en présence de fixateurs de radicaux.

10. Procédé selon la revendication 1, **caractérisé en ce que** l'on réalise la réaction de la seconde étape de réaction de manière semicontinue.

11. Procédé selon la revendication 1, **caractérisé en ce que** l'on réalise la réaction de 3-diméthylaminopropionitrile avec Cl₂ dans la première étape de réaction de telle sorte que le 3-diméthylaminopropionitrile est dissout dans la forme de son produit d'addition avec HCI.

12. Produit de cristallisation avec une taille moyenne de cristaux ≤ 10 µm contenant le composé de la formule Ia et/ou IIa

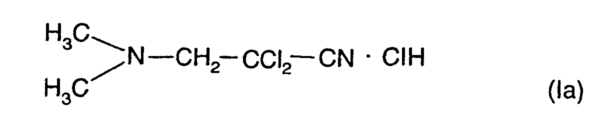

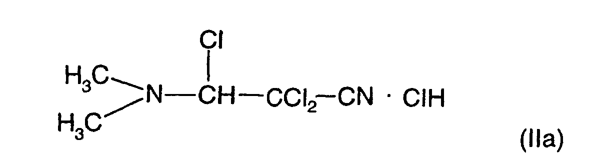

13. Procédé pour la préparation du produit de cristallisation selon la revendication 12, **caractérisé en ce que** l'on fait réagir du 3-diméthyiaminopropionltrile dans un solvant inerte par rapport à HCI et Cl₂ à une température de -10 à 55°C avec de 1 à 6 mol de HCI et de 2 à 4 mol de Cl₂, rapporté à chaque fois à 1 mol de 3-diméthylaminopropionitrile, et **en ce que** l'on maintient ensuite le mélange réactionnel ainsi obtenu à une température de 65 à 85°C.
